# FASCICULE DE BREVET EUROPEEN

(11) **EP 0 957 938 B1**
(45) Date de publication et mention de la délivrance du brevet: **01.08.2001**
(21) Numéro de dépôt: 97903159.8
(22) Date de dépôt: 14.02.1997
(51) Int. Cl.: A61K 47/10, A61K 47/14, A61K 47/44

(54) **COMPOSITION A BASE D'HUILE DE COCO ET SON UTILISATION**
ZUBEREITUNG AUF DER BASIS VON KOKOSÖL UND IHRE VERWENDUNG
COMPOSITION WITH BASE OF COCONUT OIL AND ITS USE

(43) Date de publication de la demande: 24.11.1999
(73) Titulaire: Streels, Elisabeth, 4480 Engis (BE)
(72) Inventeur: Streels, Elisabeth, 4480 Engis (BE)
(74) Mandataire: Powis de Tenbossche, Roland
(86) Numéro de dépôt international: BE9700016
(87) Numéro de publication internationale: WO9835700

(56) Documents cités:
- WO-A-91/01717
- US-A- 5 104 656
- US-A- 5 472 982

## Description

La présente invention a pour objet une composition contenant de l'alcool cétylique, de l'huile de coco, éventuellement de l'eau, et éventuellement d'autres additifs et/ou principe(s) pharmaceutiquement actif(s).

On connaît par le document WO 91/01717 une composition grasse à base d'alcool cétylique, d'une fraction d'huile de coco, et d'autres additifs. Cette composition est apte à être mélangée à une grande quantité d'une phase aqueuse.

Toutefois cette composition n'est pas apte à être mélangée à des huiles essentielles ou à des préparations à base de paraffine ou de propylène glycol. De plus la phase aqueuse de ce document doit contenir des additifs particuliers et doit être chauffée avant d'être mélangée à la phase grasse.

La présente invention a pour objet une composition contenant avantageusement un ou des principes actifs et avantageusement exempte d'eau, qui est apte à être mélangée à de l'eau avec ou sans additifs, pour former une crème ou une lotion stable. La composition suivant l'invention est de plus très stable dans le temps et permet d'éviter des dégradations du principe actif par contact avec de l'eau lors du stockage de la composition. Ainsi, la composition suivant l'invention permet de reformer une crème ou une lotion contenant le principe actif juste avant son emploi.

Enfin, la composition suivant l'invention en particulier lorsqu'elle est sensiblement exempte d'eau présente une excellente résistance contre le rancissement, même si elle contient de l'huile de coco entière, et non une fraction particulière de l'huile de coco connue sous le nom de Miglyol, fraction connue pour présenter une meilleure résistance au rancissement que l'huile entière.

La composition suivant l'invention contient un additif particulier à savoir du glycéride polyoxyéthyléné, c'est-à-dire le produit de l'alcoolyse d'une huile -végétale naturelle par des polyoxyéthylène glycols. Ledit additif est en particulier un glycéride oléo-linoléique polyoxyéthyléné ou le produit de l'alcoolyse de l'huile de maïs naturelle par des polyoxyéthylène glycols.

La composition suivant l'invention est donc une composition contenant de l'alcool cétylique, de l'huile de coco, du glycéride oléo-linoléique polyoxyéthyléné, éventuellement de l'eau, et éventuellement d'autres additifs et/ou principe(s) pharmaceutiquement actif(s), ladite composition contenant en % de matière sèche :
de 5 à 15 % de glycéride oléo-linoléique polyoxyéthylène, et
de 20 à 40% d'alcool cétylique,
le rapport en poids huile de coco et autre(s) additifs et principe(s) actif(s)/alcool cétylique étant compris entre 2/1 et 80/15. Ce rapport en poids est important pour obtenir une bonne stabilité de la composition, en particulier après ajout d'une quantité importante d'eau.

De préférence, le rapport en poids huile de coco et autre(s) additifs et principe(s) actif(s)/alcool cétylique est compris entre 2/1 et 70/25. De façon particulièrement avantageuse, ce rapport est compris entre 2,1 et 2,45.

Selon une forme de réalisation, la composition suivant l'invention contient en % de matière sèche :
de 5 à 10 % de glycéride oléo-linoléique polyoxyéthyléné, et
de 25 à 35% d'alcool cétylique.

De façon avantageuse, la composition suivant l'invention est conditionnée sans eau ou sensiblement sans eau, l'eau nécessaire à son utilisation étant ajouté juste avant son utilisation. La composition contient par exemple moins de 5% en poids d'eau et se présente avantageusement sous la forme d'une poudre apte à être mélangée à une phase aqueuse pour former un lait ou une crème. De préférence, cette poudre présente une granulométrie inférieure à 500 µm, de préférence inférieur à 100 µm,

Selon une forme de réalisation, la composition contient de 5 à 95 % en poids d'eau, par exemple de 5 à 50 % en poids d'eau.

La composition suivant l'invention contient avantageusement un ou des principes thérapeutiquement actifs, par exemple sous forme d'huile essentielle, d'extrait alcoolique ou hydro-alcoolique, de chlohexidri gluconas, d'extrait fluide, de préparation à base de propylèneglycol, de préparation à base de paraffine, de préparation à base de macérât glycériné, ou d'un mélange de ceux-ci.

La composition peut contenir, en % de matière sèche, jusqu'à 75 % de principe thérapeutiquement actif ou de principes thérapeutiquement actifs.

Etant donné que la composition suivant l'invention peut se présenter sous la forme d'une poudre et que le ou les principes actifs sont avantageusement distribués de manière homogène dans la poudre, la composition peut être conditionné sous forme de sachet contenant une dose de principe(s) actif(s). Ceci permet d'assurer que le patient après avoir préparé sa crème ou lait reçoit la dose appropriée pour son traitement.

L'invention a encore pour objet un procédé de préparation d'une poudre contenant au moins un principe thérapeutiquement actif, ladite poudre étant destinée à être mélangée à un milieu aqueux pour former une crème ou un lait. Dans ce procédé :
* on mélange sous forme liquide (par exemple après chauffage et fusion des produits sous forme solide à la température ambiante) :
   de l'alcool cétylique, de l'huile de coco, du glycéride oléo-linoléique polyoxyéthyléné, un ou des principe(s) pharmaceutiquement actif(s), et éventuellement d'autres additifs , de manière à former un mélange contenant moins de 5% d'eau
* dans lequel on transforme ledit mélange en une poudre, et
* dans lequel on mélange ladite poudre avec un ou des principe(s) pharmaceutiquement actif(s), et éventuellement avec d'autres additifs.

Selon une forme de réalisation, on transforme le mélange en poudre après refroidissement et solidification du mélange.

Selon une autre forme de réalisation possible, on transforme le mélange en poudre par pulvérisation du mélange dans une atmosphère à une température inférieure à la température de solidification.

De façon avantageuse, on ajuste la quantité d'huile de coco de manière à ce que le rapport en poids huile de coco et autre(s) additifs et principe(s) actif(s)/alcool cétylique soit compris entre 70/25 et 2/1.

Selon des formes de réalisation, on utilise une quantité de glycéride oléo-linoléique polyoxyéthyléné et d'alcool cétylique telle que la poudre contient en % de matière sèche :
de 5 à 10 % de glycéride oléo-linoléique polyoxyéthyléné, et
de 25 à 35% d'alcool cétylique.

Selon une forme de réalisation, on transforme le mélange en une poudre présentant une granulométrie inférieure à 500 µm, de préférence inférieure à 100 µm, par exemple inférieure à 50 µm.

De préférence, on prépare dans une première étape, sous forme liquide, un mélange contenant :
de l'alcool cétylique, de l'huile de coco, du glycéride oléo-linoléique polyoxyéthyléné, et éventuellement un ou des additifs, et dans une deuxième étape, on ajoute au mélange liquide préparé dans la première étape, un ou des principe(s) pharmaceutiquement actif(s).

On aurait également pu préparer, dans une première étape, sous forme liquide, un mélange contenant :
de l'alcool cétylique, et de l'huile de coco, et éventuellement un ou des additifs, et, ans une deuxième étape, ajouter au mélange liquide préparé dans la première étape, un ou des principe(s) pharmaceutiquement actif(s) mélangé à du glycéride oléo-linoléique polyoxyéthyléné.

Par exemple, on peut utiliser un ou des principes thérapeutiquement actifs, sous forme d'huile essentielle, d'extrait alcoolique ou hydro-alcoolique, de chlohexidri gluconas, d'extrait fluide, de préparation à base de propylèneglycol, de préparation à base de paraffine, de préparation à base de macérât glycériné, ou d'un mélange de ceux-ci.

Des exemples de réalisation seront décrits ci-après.

Des mélanges suivant l'invention ont été préparés en mélangeant sous forme liquide de l'huile de coco, du labrafil (glycéride oléo-linoléique polyoxyéthyléné, huile de maïs ), de l'alcool cétylique, éventuellement de la glycérine.

Après refroidissement du mélange à température ambiante, on a transformé le mélange en une poudre.

Le tableau suivant reprend les parts en poids des différents composés utilisés pour la préparation des mélanges :

| mélange | 1 | 2 | 3 | 4 | 5 | 6 | 7 |
|---|---|---|---|---|---|---|---|
| Huile de coco | 60 | 52 | 60 | 60 | 40 | 55 | 55 |
| alcool cétylique | 25 | 27 | 25 | 30 | 25 | 27 | 30 |
| labrafil | 5 | 6 | 10 | 7 | 6 | 8 | 5 |
| glycérine | | | | | 19 | 5 | |

La poudre avait une granulométrie d'environ 50 µm.

Les poudres des mélanges 1 à 7 ont été mélangées à des huiles essentielles, à savoir un mélange contenant 4,5 part en poids de Romarin, 6 pars en poids de Wintergreen, 6 parts en poids de sauge scaldé et 7, 5 parts en poids de camphre.

Ces poudres ont montrées une excellente stabilité dans le temps.

La composition des poudres ainsi préparées est donnée en part en poids dans le tableau suivant :

| Poudre | 1 | 2 | 3 | 4 | 5 | 6 | 7 |
|---|---|---|---|---|---|---|---|
| Huile de coco | 60 | 52 | 60 | 60 | 40 | 55 | 55 |
| alcool cétylique | 25 | 27 | 25 | 30 | 25 | 27 | 30 |
| labrafil | 5 | 6 | 10 | 7 | 6 | 8 | 5 |
| glycérine | | | | | 19 | 5 | |
| huiles essentielles | 5 | 15 | 5 | 3 | 10 | 5 | 10 |
| Rapport H/A | 65/25 | 67/27 | 65/25 | 63/30 | 69/25 | 65/27 | 65/30 |
| Rapport H/A: rapport en poids huile de coco et autre(s) additifs et principe(s) actif(s)/alcool cétylique | | | | | | | |

Les poudres ainsi préparées ont ensuite été mélangées à température ambiante à de l'eau pour former soit une crème, soit un lait ou une lotion. En ajoutant une part en poids d'eau par part en poids de poudre, on a obtenu une crème, tandis qu'en ajoutant 5 parts en poids d'eau par part en poids de poudre on a obtenu une lotion.

Des tests ont montrés que les crèmes ainsi préparées n'étaient pas trop grasses, n'étaient pas irritantes, et que la formation de croûtes pouvait être évitée.

Les crèmes préparées avait une texture sensiblement équivalente à celle du produit Nivéa, et ce malgré la présence d'une importante quantité d'eau.

Les crèmes préparées par mélange d'une part en poids de poudre et d'une part en poids d'eau ont été mélangées à raison de 0,1 part en poids d'un agent antibiotique pour 1 part en poids de crème ou à raison de 1 part en poids de "Merfen"® pour 1 part en poids de crème.

La poudre obtenue à partir du mélange 2 a été mélangée aux mélanges d'huiles essentielles suivantes:
Mélange d'huiles essentielles n° 1 : Romarin 3g, Wintergreen 5,5g, Camphre 4,5g, et Sauge 1 g, ce mélange d'huile essentielle a été mélangée à 86 g de poudre du mélange 2.
Mélange d'huiles essentielles n° 2 : Romarin 4,4g, Wintergreen 6,8g, Betula 2,8 g, et Laurus nobilis 2,8 g, ce mélange d'huile essentielle a été mélangée à 83,2g de poudre du mélange 2.
Mélange d'huiles essentielles n° 3 Calendula 1,2g, Alcool à 70% 2,5g, Romarin 1,5g, Lavande 5,5 g, Thym 1,5g et Sauge 1,5 g, ce mélange d'huile essentielle a été mélangée à 86,3 g de poudre du mélange 2.
Mélange d'huiles essentielles n° 4 : Calendula 1,5g, Thym 5g, Lavande 5g, Néomycine 500.000 U, ce mélange d'huiles essentielles a été mélangée à 88,5g de poudre du mélange 2.

Les compositions ainsi obtenues avaient des propriétés antibiotiques et désinfectantes. Des tests ont semblé montrer une meilleure efficacité du traitement.

De façon similaire, on a préparé des poudres contenant en tant que principes actifs d'autres huiles essentielles, des extraits alcooliques ou hydro-alcooliques, de chlohexidri gluconas, des extraits fluides, des préparations à base de propylèneglycol, de préparation à base de paraffine, des préparations à base de macérât glycériné, ou des mélanges de ceux-ci. La poudre suivant l'invention peut contenir jusqu'à 15% en poids de principes actifs, voire beaucoup plus. Avant son utilisation, la poudre est transformée en crème ou lotion. La poudre est avantageusement mise sous forme de sachet contenant une dose de principe actif. Dans ce cas, le patient est apte à préparer une crème ou lotion contenant juste la dose de principes actifs qui doit être appliquée sur son corps ou partie de corps.

La composition suivant l'invention, en particulier sous forme de crème pour application sur la peau, contient éventuellement d'autres alcools, tels que de l'éthanol, du propanol, de l'isopropanol.

Il va de soi que la composition selon l'invention peut en outre contenir un agent tensioactif, avantageusement un tensioactif anionique, de préférence un excipient huileux (tel qu'une huile ) et/ou un principe actif pharmaceutique et/ou cosmétique, par exemple un agent antibiotique, et/ou une ou des huiles essentielles, et/ou un ou des parfums..... Par exemple, la composition contient un agent pénétrant et/ou un agent révulsif et/ou un agent vasodilatateur et/ou un agent analgésique.

L'invention a également pour objet une pommade, crème ou préparation visqueuse contenant une composition suivant l'invention, un bâtonnet ou produit solide contenant une composition suivant l'invention, un support (par exemple poreux) muni d'une couche de composition suivant l'invention. un doigt, gant ou partie de gant muni sur sa paroi intérieure d'une couche d'une composition suivant l'invention.

L'invention a encore pour objet l'utilisation d'une composition suivant l'invention pour la préparation de compositions présentant une efficacité accrue d'un principe actif appliqué sur la peau d'un patient ou d'un animal.

Un autre objet de l'invention est un kit comprenant un premier récipient contenant une composition suivant l'invention et un deuxième récipient contenant un principe actif ou un agent cosmétique, pour l'application simultanée ou l'un après l'autre de la composition et du principe ou agent, ou vice versa.

Toujours un autre objet de l'invention est une préparation pharmaceutique pour le traitement de la peau à titre curatif et/ou préventif, cette préparation contenant une composition suivant l'invention.

L'invention a donc également pour objet l'utilisation de l'huile de coco en tant qu'agent actif dans la préparation de compositions pour la cicatrisation de plaies, de compositions vasodilatatrices, de compositions anti-douleur.

Lors de l'élaboration des compositions suivant l'invention, on a remarqué que pour traiter des maladies ou troubles, tels que l'arthrose, il était avantageux d'utiliser en combinaison un agent pénétrant et des autres agents vasodilatateur, analgésique, anti inflammatoire, décongestionnant et révulsif, en particulier d'utiliser en combinaison les huiles essentielles suivantes : le Romarin, le Wintergreen, la sauge (scaldée) et le camphre. L'invention a donc encore pour objet des compositions comprenant les huiles essentielles décrites ci-avant, compositions convenant pour le traitement de la peau, de l'arthrose, ... Ces compositions comprennent de façon avantageuse une composition du type décrit à la revendication 1, mais peuvent contenir d'autres excipients.

## Revendications

1. Composition contenant de l'alcool cétylique, de l'huile de coco, du glycéride oléo-linoléique polyoxyéthyléné, éventuellement de l'eau, et éventuellement d'autres additifs et/ou principe(s) pharmaceutiquement actif(s), ladite composition contenant en % de matière sèche :
de 5 à 15 % de glycéride oléo-linoléique polyoxyéthyléné, et
de 20 à 40% d'alcool cétylique,
le rapport en poids huile de coco et autre(s) additifs et principe(s) actif(s)/alcool cétylique étant compris entre 2/1 et 80/15.

2. Composition suivant la revendication 1, caractérisée en ce que le rapport en poids huile de coco et autre(s) additifs et principe(s) actif(s)/alcool cétylique est compris entre 2/1 et 70/25.

3. Composition suivant la revendication 1 ou 2. caractérisée en ce qu'elle contient en % de matière sèche :
de 5 à 10 % de glycéride oléo-linoléique polyoxyéthyléné, et
de 25 à 35% d'alcool cétylique.

4. Composition suivant l'une des revendications 1 à 3, caractérisée en ce qu'elle contient moins de 5% en poids d'eau et en ce qu'elle se présente sous la forme d'un bloc pouvant être mis sous forme de poudre, mais de préférence sous forme d'une poudre apte à être mélangée à une phase aqueuse pour former un lait ou une crème.

5. Composition suivant la revendication 4, caractérisée en ce que la poudre présente une granulométrie inférieure à 500 µm, de préférence inférieure à 100 µm.

6. Composition suivant l'une des revendications 1 à 3, caractérisée en ce qu'elle contient de 5 à 95 % en poids d'eau.

7. Composition suivant la revendication 6, caractérisée en ce qu'elle contient de 5 à 50 % en poids d'eau.

8. Composition suivant l'une quelconque des revendications 1 à 7, caractérisée en ce qu'elle contient un ou des principes thérapeutiquement actifs, sous forme d'huile essentielle, d'extrait alcoolique ou hydro-alcoolique, de chlohexidri gluconas, d'extrait fluide, de préparation à base de propylèneglycol, de préparation à base de paraffine, de préparation à base de macérat glycériné, ou d'un mélange de ceux-ci.

9. Composition suivant l'une quelconque des revendications 1 à 8, caractérisée en ce qu'elle contient en % de matière sèche jusqu'à 15 % de principe thérapeutiquement actif ou de principes thérapeutiquement actifs.

10. Procédé de préparation d'une poudre selon la revendication 4 contenant au moins un principe thérapeutiquement actif, ladite poudre étant destinée à être mélangée à un milieu aqueux pour former une crème ou un lait,
* dans lequel on mélange sous forme liquide :
de l'alcool cétylique, de l'huile de coco, du glycéride oléo-linoléique polyoxyéthyléné. de manière à former un mélange contenant moins de 5% d'eau.
* dans lequel on transforme ledit mélange en une poudre, et
* dans lequel on mélange ladite poudre avec un ou des principe(s) pharmaceuliquement actif(s), et éventuellement avec d'autres additifs.

11. Procédé suivant la revendication 10, caractérisé en ce qu'on transforme le mélange en poudre après refroidissement et solidification du mélange.

12. Procédé suivant la revendication 10, caractérisé en ce qu'on transforme le mélange en poudre par pulvérisation du mélange dans une atmosphère à une température inférieure à la température de solidification.

13. Procédé suivant l'une des revendications 10 à 12, caractérisé en ce qu'on ajuste la quantité d'huile de coco utilisé pour que la poudre présente un rapport en poids huile de coco et autre(s) additifs et principe(s) actif(s)/alcool cétylique entre 70/25 et 2/1.

14. Procédé suivant l'une des revendications 10 à 12, caractérisé en ce qu'on utilise une quantité de glycéride oléo-linoléique polyoxyéthyléné et d'alcool cétylique telle que la poudre contient en % de matière sèche :
de 5 à 10 % de glycéride oléo-linoléique polyoxyéthyléné, et
de 25 à 35% d'alcool cétylique.

15. Procédé suivant l'une des revendications 10 à 14, caractérisé en ce qu'on transforme le mélange en une poudre présentant une granulométrie inférieure à 500 µm, de préférence inférieur à 100 µm.

16. Procédé suivant l'une des revendications 10 à 15, caractérisé en ce qu'on prépare dans une première étape, sous forme liquide. un mélange contenant :
de l'alcool cétylique, de l'huile de coco, du glycéride oléo-linoléique polyoxyéthyléné, et éventuellement un ou des additifs, et en ce que dans une deuxième étape, on ajoute au mélange liquide préparé dans la première étape, un ou des principe(s) pharmaceutiquement actif(s) sous forme liquide.

17. Procédé suivant l'une des revendications 10 à 15, caractérisé en ce qu'on prépare dans une première étape, sous forme liquide, un mélange contenant :
de l'alcool cétylique, et de l'huile de coco, et éventuellement un ou des additifs, et en ce que dans une deuxième étape, on ajoute au mélange liquide préparé dans la première étape, un ou des principe(s) pharmaceutiquement actif(s) prémélangé à du glycéride oléo-linoléique polyoxyéthyléné.

18. Procédé suivant la revendication 16 ou 17, caractérisé en ce qu'on utilise un ou des principes thérapeutiquement actifs, sous forme d'huile essentielle, d'extrait alcoolique ou hydro-alcoolique, de chlohexidri gluconas, d'extrait fluide, de préparation à base de propylèneglycol, de préparation à base de paraffine, de préparation à base de macérât glycériné, ou d'un mélange de ceux-ci.

## Claims

1. Composition containing cetyl alcohol, coconut oil, polyoxyethylene oleo-linoleic glyceride, possibly water, and possibly other additives and/or pharmaceutically active principle(s), said composition containing in % of dry matter :
5 to 15 % polyoxyethylene oleo-linoleic glyceride, and
20 to 40 % cetyl alcohol,
the weight ratio coconut oil and other additives and/or pharmaceutically active principle(s)/cetyl alcohol being comprised between 2/1 and 80/15.

2. Composition according to claim 1, characterized in that the weight ratio coconut oil and other additives and/or pharmaceutically active principle(s)/cetyl alcohol is comprised between 2/1 and 70/25.

3. Composition according to claim 1 or 2, characterized in that it contains in % of dry matter:
5 to 10 % polyoxyethylene oleo-linoleic glyceride, and
20 to 35 % cetyl alcohol.

4. Composition according to one of the claims 1 to 3, characterized in that it contains less than 5 % by weight water and in that it has the form of a mass suitable to be put in powder form, but preferably in the form of a powder suitable to be mixed with an aqueous phase for forming a milk or cream.

5. Composition according to claim 4, characterized in that the powder has a particle size lower than 500µm, preferably lower than 100µm.

6. Composition according to one of the claims 1 to 3, characterized in that it contains 5 to 95 % by weight water.

7. Composition according to claim 6 characterized in that it contains 5 to 50 % by weight water.

8. Composition according to anyone of the claims 1 to 7, characterized in that it contains one or more pharmaceutically active principles, in the form of essential oil, alcohol extract or hydro-alcohol extract, chlohexidri gluconas, liquid extract, preparation containing propyleneglycol, preparation containing paraffin, preparation containing glycerin soaking product, or a mixture thereof.

9. Composition according to anyone of the claims 1 to 8, characterized in that it contains in % of dry matter, up to 15% of pharmaceutically active principle(s).

10. Process for the preparation of a powder according to claim 4 containing at least a pharmaceutically active principle, the said powder being intended to be mixed with an aqueous medium for giving a cream or a milk,
• in which cetyl alcohol, coconut oil, polyoxyethylene oleo-linoleic glyceride are mixed in liquid form so as to form a mixture containing less than 5 % water,
• in which the said mixture is transformed into a powder, and
• in which the said powder is mixed with one or more pharmaceutically active agents, and possibly with other additives.

11. Process according to claim 10, characterized in that the mixture is transformed into a powder after cooling and solidification of the mixture.

12. Process according to claim 10, characterized in that the mixture is transformed into a powder by spraying the mixture in an atmosphere, the temperature of which is lower than the solidification temperature.

13. Process according to one of the claims 10 to 12, characterized in that the used amount of coconut oil is adjusted so that the powder has a weight ratio coconut oil and other additives and/or pharmaceutically active principle(s)/cetyl alcohol comprised between 70/25 and 2/1.

14. Process according to one of the claims 10 to 12, characterized in that polyoxyethylene oleo-linoleic glyceride and cetyl alcohol are used in an amount such that the powder contains in % of dry matter :
5 to 10 % polyoxyethylene oleo-linoleic glyceride, and
20 to 35 % cetyl alcohol.

15. Process of one of the claims 10 to 14, characterized in that the mixture is transformed into a powder with a particle size lower than 500µm, preferably lower than 100µm.

16. Process according to one of the claims 10 to 15, characterized in that, in a first step, a liquid mixture containing cetyl alcohol, coconut oil, and possibly one or more additives is prepared, and in that in a second step, one or more pharmaceutically active principles are added in liquid form to the liquid mixture prepared in the first step.

17. Process according to one of the claims 10 to 15, characterized in that in a first step, a liquid mixture containing cetyl alcohol, coconut oil, polyoxyethylene oleo-linoleic glyceride, and possibly one or more additives is prepared, and in that in a second step, one or more pharmaceutically active principles premixed with polyoxyethylene oleo-linoleic glyceride, are added to the liquid mixture prepared in the first step.

18. Process according to claim 16 or 17, characterized in that contains one or more pharmaceutically active principles, in the form of essential oil, alcohol extract or hydro-alcohol extract, chlohexidri gluconas, liquid extract, preparation containing propyleneglycol, preparation containing paraffin, preparation containing glycerin soaking product, or a mixture thereof, are used.

## Patentansprüche

1. Zusammensetzung, enthaltend Cetylalkohol, Kokosöl, Oleyl-Linoleyl-Polyoxyethylenglycerid, gegebenenfalls Wasser, und gegebenenfalls eines oder mehrere andere Additive und/oder einen oder mehrere pharmazeutische Wirkstoffe, wobei die Zusammensetzung in Prozent der Trockenmasse
von 5 bis 15 % Oleyl-Linoleyl-Polyoxyethylenglycerid, und
von 20 bis 40 % Cetylalkohol enthält, und
das Gewichtsverhältnis von Kokosöl und anderen Additiven und einem oder mehreren Wirkstoffen zu Cetylalkohol von 2:1 bis 80:15 beträgt.

2. Zusammensetzung nach Anspruch 1, dadurch gekennzeichnet, daß das Gewichtsverhältnis von Kokosöl und dem einen oder mehreren anderen Additiven und einem oder mehreren Wirkstoffen zu Cetylalkohol von 2:1 bis 70:25 beträgt.

3. Zusammensetzung nach Anspruch 1 oder 2, dadurch gekennzeichnet, daß sie, in Prozent der Trockenmasse,
von 5 bis 10 % Oleyl-Linoleyl-Polyoxyethylenglycerid, und
von 25 bis 35 % Cetylalkohol enthält.

4. Zusammensetzung nach einem der Ansprüche 1 bis 3, dadurch gekennzeichnet, daß sie weniger als 5 Gewichtsprozent Wasser enthält und in Form eines Feststoffes vorliegt, welcher in ein Pulver überführt werden kann, aber bevorzugt in ein Pulver, welches zum Mischen mit einer wässrigen Phase um eine Milch oder Creme zu bilden, geeignet ist.

5. Zusammensetzung nach Anspruch 4, dadurch gekennzeichnet, daß das Pulver eine Korngröße kleiner 500 µm, bevorzugt kleiner 100 µm aufweist.

6. Zusammensetzung nach einem der Ansprüche 1 bis 3, dadurch gekennzeichnet, daß sie 5 bis 95 Gewichtsprozent Wasser enthält.

7. Zusammensetzung nach Anspruch 6, dadurch gekennzeichnet, daß sie 5 bis 50 Gewichtsprozent Wasser enthält.

8. Zusammensetzung nach einem der Ansprüche 1 bis 7, dadurch gekennzeichnet, daß sie einen oder mehrere therapeutische Wirkstoffe in Form eines etherischen Öls, eines Alkoholextrakts oder wässrigen Alkoholextrakts, von Chlorhexidinum Gluconicum, eines fluiden Extrakts, einer Präparation auf Basis von Propylenglykol, einer Präparation auf Basis von Paraffin, einer Präparation auf Basis von glyzerinhaltigem zerkleinerten Material, oder eines Gemisches davon enthält.

9. Zusammensetzung nach einem der Ansprüche 1 bis 8, dadurch gekennzeichnet, daß sie bis zu 15% eines oder mehrerer therapeutischer Wirkstoffe, bezogen auf Prozent Trockenmasse, enthält.

10. Verfahren zur Herstellung eines Pulvers nach Anspruch 4, welches mindestens einen therapeutischen Wirkstoff enthält, wobei das Pulver dazu vorgesehen ist, in ein wässriges Medium gemischt zu werden um eine Creme oder eine Milch zu bilden, wobei man
- Cetylalkohol, Kokosöl, Oleyl-Linoleyl-Polyoxyethylenglycerid in flüssiger Form derart vermischt, so daß ein Gemisch gebildet wird, das weniger als 5% Wasser enthält,
- das Gemisch in ein Pulver überführt, und
- das Gemisch mit einem oder mehreren pharmazeutischen Wirkstoffen und gegebenenfalls anderen Additiven vermischt.

11. Verfahren nach Anspruch 10, dadurch gekennzeichnet, daß man das Gemisch nach Abkühlen und Festwerden des Gemisches in ein Pulver überführt.

12. Verfahren nach Anspruch 10, dadurch gekennzeichnet, daß man das Gemisch in einer Atmosphäre bei einer Temperatur unterhalb der Erstarrungstemperatur des Gemisches in ein Pulver überführt.

13. Verfahren nach einem der Ansprüche 10 bis 12, dadurch gekennzeichnet, daß man die verwendete Menge an Kokosöl derart einstellt, so daß das Pulver ein Gewichtsverhältnis von Kokosöl und einem oder mehreren anderen Additiven und einem oder mehreren Wirkstoffen zu Cetylalkohol zwischen 70:25 und 2:1 aufweist.

14. Verfahren nach einem der Ansprüche 10 bis 12, dadurch gekennzeichnet, daß man eine derartige Menge an Oleyl-Linoleyl-Polyoxyethylenglycerid und Cetylalkohol verwendet, so daß das Pulver in Prozent der Trockenmasse
von 5 bis 10 % Oleyl-Linoleyl-Polyoxyethylenglycerid, und
von 25 bis 35 % Cetylalkohol enthält.

15. Verfahren nach einem der Ansprüche 10 bis 14, dadurch gekennzeichnet, daß man das Gemisch in ein Pulver überführt, welches eines Korngröße kleiner 500 µm, bevorzugt kleiner 100 µm aufweist.

16. Verfahren nach einem der Ansprüche 10 bis 15, dadurch gekennzeichnet, daß man in einem ersten Schritt ein Gemisch in flüssiger Form, welches Cetylalkohol, Kokosöl, Oleyl-Linoleyl-Polyoxyethylenglycerid und gegebenenfalls eines oder mehrere Additive enthält, herstellt, und in einem zweiten Schritt dem im ersten Schritt hergestellten Gemisch einen oder mehrere pharmazeutische Wirkstoffe in flüssiger Form zugibt.

17. Verfahren nach einem der Ansprüche 10 bis 15, dadurch gekennzeichnet, daß man in einem ersten Schritt ein Gemisch in flüssiger Form, welches Cetylalkohol und Kokosöl und gegebenenfalls eines oder mehrere Additive enthält, herstellt, und in einem zweiten Schritt dem im ersten Schritt hergestellten Gemisch einen oder mehrere, in Oleyl-Linoleyl-Polyoxyethylenglycerid vorgemischte pharmazeutische Wirkstoffe zugibt.

18. Verfahren nach Anspruch 16 oder 17, dadurch gekennzeichnet, daß man einen oder mehrere therapeutische Wirkstoffe in Form eines etherischen Öls, eines Alkoholextrakts oder wässrigen Alkoholextrakts, von Chlorhexidinum Gluconicum, eines fluiden Extrakts, einer Präparation auf Basis von Propylenglykol, einer Präparation auf Basis von Paraffin, einer Präparation auf Basis von glyzerinhaltigem zerkleinerten Material, oder eines Gemisches davon verwendet.
